(19) Europäisches Patentamt
European Patent Office
Office européen des brevets

(11) Veröffentlichungsnummer: **0 032 199**
**A1**

(12) # EUROPÄISCHE PATENTANMELDUNG

(21) Anmeldenummer: **80107694.4**

(22) Anmeldetag: **06.12.80**

(51) Int. Cl.$^3$: **C 07 D 233/90**
C 07 D 487/14, A 01 N 43/50

(30) Priorität: **19.12.79 DE 2951201**

(43) Veröffentlichungstag der Anmeldung:
**22.07.81 Patentblatt 81 29**

(84) Benannte Vertragsstaaten.
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Beck, Gunther, Dr.**
**Am Mittelberg 19**
**D-5090 Leverkusen(DE)**

(72) Erfinder **Heitzer, Helmut, Dr.**
**Höhenstrasse 84**
**D-5090 Leverkusen(DE)**

(72) Erfinder **Lürssen, Klaus, Dr.**
**August-Kierspel-Strasse 89**
**D-5070 Bergisch-Gladbach(DE)**

(54) **Mittel zur Regulierung des Pflanzenwachstums, deren Herstellung und deren Verwendung.**

(57) Teilweise bekannte Imidazol-2-carbonsäureamide der Formel

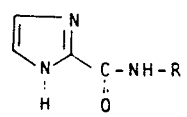

(I)

in welcher
R für Alkyl steht,

besitzen starke pflanzenwuchsregulierende Eigenschaften.

Die Stoffe dep Formel (I), in denen R für Alkyl, jedoch nicht für n-Butyl steht, sind neu. Sie lassen sich nach verschiedenen Verfahren herstellen.

EP 0 032 199 A1

BAYER AKTIENGESELLSCHAFT
Zentralbereich
Patente Marken und Lizenzen

5090 Leverkusen-Bayerwerk
Dü/ABc
IIa

Mittel zur Regulierung des Pflanzenwachstums, deren Herstellung und deren Verwendung

Die vorliegende Erfindung betrifft die Verwendung von teilweise bekannten Imidazol-2-carbonsäureamiden als Wirkstoffe zur Regulierung des Pflanzenwachstums.

Es ist bereits bekannt geworden, daß sich bestimmte Imidazol-2-carbonsäureamide durch Umsetzung von Imidazol mit Isocyanaten herstellen lassen (vgl. J. Org. Chem. 1977 (42), 3925). So entsteht zum Beispiel bei der Reaktion von Imidazol mit n-Butylisocyanat in siedendem Nitrobenzol das Imidazol-2-carbonsäure-n-butylamid.

Außerdem ist bereits bekannt geworden, daß bestimmte 4,5-Dichlorimidazol-2-carbonsäureamide herbizide Eigenschaften besitzen (vgl. DE-OS 26 10 527). So kann zum Beispiel das 4,5-Dichlorimidazol-2-carbonsäure-ethylamid zur Bekämpfung von Unkraut eingesetzt werden. Eine Verwendung dieser Stoffe zur Regulierung des Pflanzenwachstums wurde jedoch bisher noch nicht beschrieben.

Es wurde jetzt gefunden, daß die teilweise bekannten Imidazol-2-carbonsäureamide der Formel

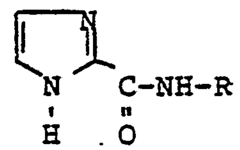
(I)

in welcher

R für Alkyl steht,

starke wachstumsregulierende Eigenschaften aufweisen.

Überraschenderweise zeigen die erfindungsgemäß verwendbaren Imidazol-2-carbonsäureamide der Formel (I) hervorragende pflanzenwachstumsregulierende Wirkung. Sie lassen sich mit besonderem Vorteil zur Wuchsförderung einsetzen, und zwar vor allem bei Zuckerrüben. Die erfindungsgemäße Verwendung der Imidazol-2-carbonsäureamide der Formel (I) stellt somit eine wertvolle Bereicherung der Technik dar.

Die erfindungsgemäß verwendbaren Imidazol-2-carbonsäureamide sind durch die Formel (I) allgemein definiert. In dieser Formel steht R vorzugsweise für geradkettiges oder verzweigtes Alkyl mit 1 bis 18 Kohlenstoffatomen.

Ganz besonders bevorzugt sind diejenigen Verbindungen der Formel (I), in denen R für Methyl, Ethyl, n-Propyl, Isopropyl, n-Butyl, i-Butyl, sek.-Butyl, tert.-Butyl,

n-Pentyl, neo-Pentyl, n-Hexyl, n-Heptyl, n-Octyl, n-Decyl, n-Dodecyl und n-Octadecyl steht.

Die erfindungsgemäß verwendbaren Imidazol-2-carbonsäureamide der Formel (I) sind teilweise bekannt (vgl. Acta Chem. Scand. 21, 279 (1967) und J. Org. Chem. 1977 (42), 3925).

Die Verbindungen der Formel

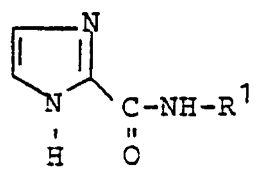

(Ia)

in welcher
$R^1$ für Alkyl, jedoch nicht für n-Butyl, steht

sind neu.

Diese neuen Stoffe der Formel (Ia) lassen sich herstellen, indem man

a) Imidazol mit Isocyanaten der Formel

$$R^1 - NCO \qquad (II)$$

in welcher

$R^1$ die oben angegebene Bedeutung hat,

in Gegenwart eines Verdünnungsmittels, wie zum Beispiel Nitrobenzol, bei Temperaturen zwischen 150°C und 250°C umsetzt, oder

b) das dimere Keten der Formel

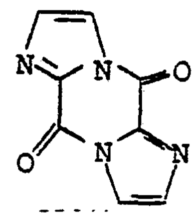

(III)

mit Aminen der Formel

$H_2N-R^1$ (IV)

in welcher

$R^1$ die oben angegebene Bedeutung hat,

gegebenenfalls in Gegenwart eines Verdünnungsmittels sowie gegebenfalls in Gegenwart eines Katalysators bei Temperaturen zwischen -50°C und +200°C umsetzt.

Die bei dem Verfahren (a) als Ausgangsstoffe benötigten Isocyanate der Formel (II) sind bekannt.

Das bei dem Verfahren (b) als Ausgangssubstanz benötigte dimere Keten der Formel (III) ist bisher noch nicht beschrieben worden. Es läßt sich herstellen, indem man Imidazol-2-carbonsäure mit einem Überschuß an Thionylchlorid gegebenenfalls in Gegenwart von Dimethylformamid als Katalysator unter Rückfluß erhitzt.

Die Imidazol-2-carbonsäure ist bekannt (vgl. Acta Chem. Scand. 21, 279 (1967)).

Als Verdünnungsmittel kommen bei der Umsetzung nach dem Verfahren (b) sowohl die als Reaktionskomponenten verwendeten Amine der Formel (IV) selbst, - sofern sie in flüssigen Zustand vorliegen oder bei niedrigen Temperaturen schmelzen -, als auch inerte organische Solventien, wie Methylenchlorid oder Chloroform, in Frage. Ferner können auch Alkohole mit 1 bis 4 Kohlenstoffatomen, wie Methanol oder Isopropanol oder auch Wasser als Verdünnungsmittel fungieren.

Als Katalysatoren können bei dem Verfahren (b) starke anorganische Basen, wie Natriumhydroxid oder Kaliumhydroxid, oder Amine, wie Triethylamin oder Triethylendiamin, verwendet werden.

Die Reaktionstemperaturen können bei dem Verfahren (b) innerhalb eines größeren Bereiches variiert werden. Im allgemeinen arbeitet man bei Temperaturen zwischen -50°C und +200°C, vorzugsweise zwischen 0°C und 100°C.

Bei der Durchführung des Verfahrens (b) setzt man auf 1 Mol an dimerem Keten oder Formel (III) 2 Mol oder auch einen größeren Überschuß an Amin der Formel (IV) sowie gegebenenfalls 0,01 bis 1, vorzugsweise 0,1 bis 0,5 Mol an Katalysator ein. Die Isolierung der entstehenden Imidazol-2-carbonsäureamide der Formel (I) erfolgt nach üblichen Methoden. Im allgemeinen verfährt man in der Weise, daß man nach beendeter Umsetzung das Lösungsmittel und/oder die im Überschuß vorhandene Komponente (IV) abdestilliert und den verbleibenden Rückstand gegebenenfalls umkristallisiert oder unter vermindertem Druck sublimiert.

In der Formel (Ia) steht $R^1$ vorzugsweise für geradkettiges oder verzweigtes Alkyl mit 1 bis 18 Kohlenstoffatomen, jedoch nicht für n-Butyl.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflußen.

Mit Wachstumsregulatoren läßt sich z.B. eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen.

Mit Wachstumsregulatoren läßt sich ferner eine Beschleunigung der Reife des Erntegutes erreichen. Dieses ist von besonderem Vorteil, weil sich dadurch eine optimale Anpassung an die Bedürfnisse des Marktes herbeiführen läßt.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,1 und 95 Gewichtsprozent Wirkstoff, vorzugsweise zwischen o,5 und 9o %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche o,o1 bis 5o kg, bevorzugt o,o5 bis 1o kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Im folgenden wird die Aktivität der erfindungsgemäß verwendbaren Stoffe als Wachstumsregulatoren anhand von Beispielen dargestellt.

Beispiel A

Wuchsförderung bei Zuckerrüben

Lösungsmittel: 30 Gewichtsteile Dimethylfomamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsförderung in Prozent des Zuwachses der Kontrollpflanzen berechnet. Es bedeuten 0 % Wuchsförderung ein Wachstum entsprechend dem der Kontrollpflanzen. Positive Werte kennzeichnen eine Wuchsförderung gegenüber den Kontrollpflanzen

Tabelle A

| Wirkstoff | Konzentration in ppm | Wuchsförderung in % |
|---|---|---|
| 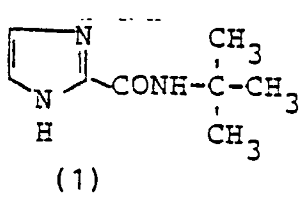 (1) | 500 | + 5 |
| | 250 | + 5 |
| | 125 | + 15 |
| | 62,5 | + 30 |
| | 31,25 | + 30 |

Tabelle A (Fortsetzung)

| Wirkstoff | Konzentration in ppm | Wuchsförderung in % |
|---|---|---|
| 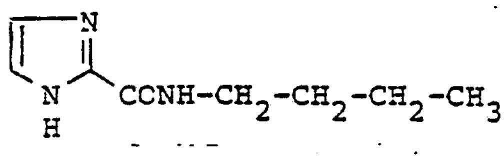 (2) | 500 | + 20 |
| 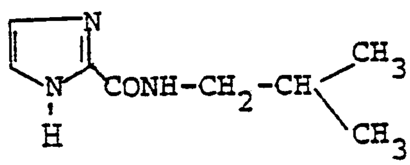 (3) | 31 | + 10 |
| - (Kontrolle) | - | 0 |

Eine Wuchsförderung junger Zuckerrübenpflanzen führt zu einem dichteren Bestand zu einem früheren Zeitpunkt während der Vegetationsperiode im Feld, was wiederum zu einer Ertragssteigerung in den Zuckerrüben führt.

Herstellungsbeispiele

Beispiel 1

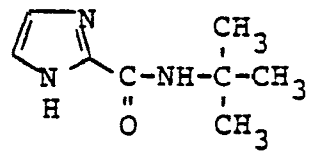

Zu einer Suspension von 188 g (1 Mol) des dimeren Ketens der Formel

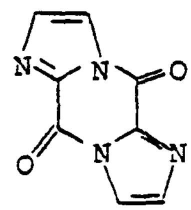

in 1,5 Liter Methylenchlorid wurden allmählich 153 g (2,1 Mol) tert.-Butylamin zugegeben, wobei die Temperatur des Reaktionsgemisches bis zum Siedepunkt (ca. 42°C) anstieg. Nach einstündigem Nachrühren unter Rückfluß wurde heiß von geringen Mengen ungelösten Bestandteilen abfiltriert und das Filtrat im Vakuum zur Trockne eingeengt. Man erhielt so das Imidazol-2-carbonsäure-tert.-butylamid in praktisch quantitativer Ausbeute. Die Verbindung ist bei 100-120°C/0,01 Torr unzersetzt sublimierbar und aus Wasser in Säulen oder aus Petroläether (Kp 40-80°C) in langen Nadeln kristallisierbar. Schmelzpunkt 134°C.

Herstellung des Ausgangsproduktes:

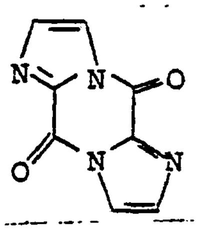

Variante α:

11,2 g (0,1 Mol) Imidazol-2-carbonsäure /herstellbar z.B. nach J. Am. Chem. Soc., 71, 383 (1949)/ wurden mit 100 ml Thionylchlorid 5 Stunden unter Rückfluß gerührt. Nach dem Erkalten wurde abgesaugt, mit etwas Petrolether gewaschen und getrocknet. Man erhielt so in fast quantitativer Ausbeute das 5H,10H-diimidazo /1,2-a:1',2'-d/pyrazin-5,10-dion in Form eines gelben Pulvers. Die Substanz schmilzt noch nicht bei 290°C. Sie ist bei 200-250°C/0,01 Torr unzersetzt in gelben Kristallen sublimierbar.
IR (KBr): 3137, 1735, 1522, 1445, 1387, 1331, 1274, 1161, 1059, 1018, 810, 800, 748, 699, 651 $cm^{-1}$.

Variante ß:

Man verfuhr wie unter α) beschrieben mit dem Unterschied, daß man 1 ml Dimethylformamid zusetzte. Die Umsetzung zur Verbindung der Formel

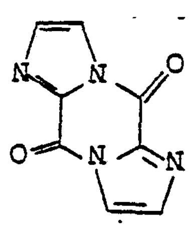

war, wie der Vergleich der IR-Spektren ergab, bereits nach etwa einer halben Stunde praktisch vollständig.

Beispiel 2

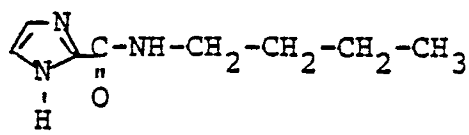

Durchführung nach J. Org. Chem. 1977 (42), 3925:

Eine Lösung von 0,10 Mol Imidazol und 0,10 Mol n-Butylisocyanat in 40 ml Nitrobenzol wurde 8 Stunden unter Rückfluß erhitzt. Nach dem Abkühlen der Reaktionsmischung wurde mit Tetrachlorkohlenstoff verdünnt, der Feststoff abgesaugt und so lange mit Tetrachlorkohlenstoff gewaschen, bis dieser farblos abläuft. Nach dem Trocknen erhielt man das Imidazol-2-carbonsäure-n-butylamid in 18 %iger Ausbeute. Schmelzpunkt 188 - 190°C.

Beispiel 3

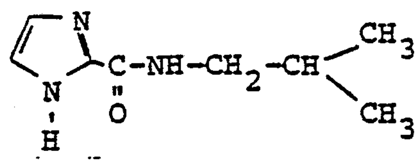

Nach der im Beispiel 2 beschriebenen Methode wurde auch das Imidazol-2-carbonsäure-isobutylamid ausgehend von Imidazol und iso-Butylisocyanat hergestellt. Schmelzpunkt: 202°C.

Nach der im Beispiel 1 angegebenen Methode wurden auch die in der nachstehenden Tabelle formelmäßig aufgeführten Verbindungen hergestellt.

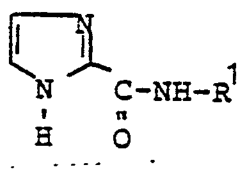

(Ia)

| Beispiel-Nr. | $R^1$ | Schmelzpunkt in °C | |
|---|---|---|---|
| 4 | $CH_3$ | 260 | (Wasser) |
| 5 | $n-C_{18}H_{37}$ | 156 | (Dioxan) |
| 6 | $C_2H_5$ | 210 | (Wasser) |
| 7 | $n-C_3H_7$ | 207 | (Acetonitril) |
| 8 | $i-C_3H_7$ | 226 | (Acetonitril) |
| 9 | $-CH_2-C(CH_3)_3$ | 184 | (Acetonitril) |
| 10 | $sec.-C_4H_9$ | 209 | (Acetonitril) |
| 11 | $n-C_6H_{13}$ | 194 | (Acetonitril) |
| 12 | 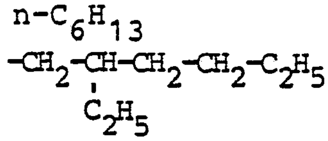 | 154 | (Dioxan) |
| 13 | $n-C_{12}H_{25}$ | 165 | (Dioxan) |
| 14 | $n-C_{14}H_{29}$ | 165 | (Acetonitril) |
| 15 | $n-C_{16}H_{33}$ | 158 | (Dioxan) |

Patentansprüche

1. Mittel zur Regulierung des Pflanzenwachstums, gekennzeichnet durch einen Gehalt an mindestens einem Imidazol-2-carbonsäureamid der Formel

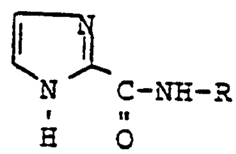

(I)

in welcher

R für Alkyl steht.

2. Mittel gemäß Anspruch 1, gekennzeichnet durch einen Gehalt an mindestens einem Imidazol-2-carbonsäureamid der Formel (I), in welcher R für geradkettiges oder verzweigtes Alkyl mit 1 bis 18 Kohlenstoffatomen steht.

3. Verfahren zur Regulierung des Pflanzenwachstums, dadurch gekennzeichnet, daß man Imidazol-2-carbonsäureamide der Formel (I) gemäß Anspruch 1 auf die Pflanzen oder ihren Lebensraum ausbringt.

4. Verwendung von Imidazol-2-carbonsäureamiden der Formel (I) gemäß Anspruch 1 zur Regulierung des Pflanzenwachstums.

5. Verfahren zur Herstellung von pflanzenwachstumsregulierenden Mitteln, dadurch gekennzeichnet, daß

man Imidazol-2-carbonsäureamide der Formel (I) gemäß Anspruch 1 mit Streckmitteln und/oder oberflächenaktiven Stoffen vermischt.

6) Imidazol-2-carbonsäureamide der Formel

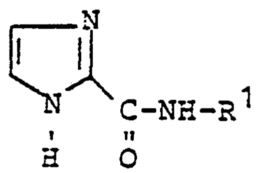

(Ia)

in welcher

$R^1$ für Alkyl, jedoch nicht für n-Butyl, steht.

7) Verfahren zur Herstellung von Imidazol-2-carbonsäureamiden der Formel (Ia), dadurch gekennzeichnet, daß man

a) Imidazol mit Isocyanaten der Formel

$$R^1\text{-NCO} \quad \text{(II)}$$

in welcher

$R^1$ die oben angegebene Bedeutung hat,

in Gegenwart eines Verdünnungsmittels umsetzt,

oder

b) das dimere Keten der Formel

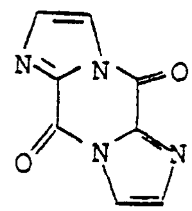

(III)

mit Aminen der Formel

$H_2N-R^1$ (IV)

in welcher

$R^1$ die oben angegebene Bedeutung hat,

gegebenenfalls in Gegenwart eines Verdünnungsmittels sowie gegebenenfalls in Gegenwart eines Katalysators umsetzt.

8) Mittel gemäß Anspruch 1, gekennzeichnet durch einen Gehalt an Imidazol-2-carbonsäure-tert.-butylamid der Formel

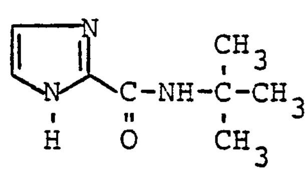

9) Imidazol-2-carbonsäure-tert.-butylamid.

10) Imidazol-2-carbonsäure-isobutylamid.

0032199

Europäisches Patentamt

# EUROPÄISCHER RECHERCHENBERICHT

Nummer der Anmeldung
EP 80 10 7694

## EINSCHLÄGIGE DOKUMENTE

| Kategorie | Kennzeichnung des Dokuments mit Angabe soweit erforderlich, der maßgeblichen Teile | betrifft Anspruch |
| --- | --- | --- |
| | DE - A - 1 921 341 (BASF)<br>* Seiten 1-4 *<br>--- | 1 |
| | DE - A - 1 567 084 (SHELL)<br>* Seiten 1-3 *<br>--- | 1 |
| | JOURNAL OF ORGANIC CHEMISTRY, Band 42, Heft 24, 25. November 1977, WASHINGTON D.C. (US) E.P. PAPADOPOULOS: "Reactions of Imidazoles with Isocyanates at Elevated Temperature", Seiten 3925-3929<br>* Seiten 3925-3928 *<br>--- | 1,7 |
| P | JOURNAL OF HETEROCYCLIC CHEMISTRY Band 17, Heft 2, März 1980, UTAH (US) J.P. DIRLAM et al.: "Syntheses and Reactions of Some 4,5-Dihaloimidazole-2-carboxylic Acid Derivatives", Seiten 409-411<br>* Seiten 409-410 *<br>--- | 1,7 |
| P | EP - A - 0 019 067 (AJINOMOTO)<br>* Seiten 1-9 *<br>--------- | 7 |

KLASSIFIKATION DER ANMELDUNG (Int Cl )

C 07 D 233/90
C 07 D 487/14
A 01 N 43/50

RECHERCHIERTE SACHGEBIETE (Int Cl )

C 07 D 233/90
A 01 N 43/50

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
| --- | --- | --- |
| Den Haag | 10.03.1981 | DE BUYSER |